# EUROPEAN PATENT SPECIFICATION

(11) **EP 2 929 341 B1**
(45) Date of publication and mention of the grant of the patent: **12.10.2016**
(21) Application number: 14780768.9
(22) Date of filing: 29.08.2014
(51) Int. Cl.: G01N 27/74

(54) **SCALABLE AND HIGH THROUGHPUT BIOSENSING PLATFORM**
SKALIERBARE BIOSENSING-PLATTFORM MIT HOHEM DURCHSATZ
PLATEFORME DE BIODÉTECTION MODULABLE ET À HAUT RENDEMENT

(30) Priority: 30.08.2013 PT 10713813
(43) Date of publication of application: 14.10.2015
(60) Divisional application: 15184108.7
(73) Proprietor: Magnomics, SA, 3060-197 Cantanhede (PT)
(72) Inventor: GERMANO, Jose Antonio Henriques Kuan, P-1000-029 Lisbon (PT); PIEDADE, Moises Simoes, P-1000-029 Lisbon (PT)
(74) Representative: Thorniley, Peter
(86) International application number: PCT/EP2014/068443
(87) International publication number: WO 2015/028642

(56) References cited:
- WO-A2-2008/075262
- LOPES P ET AL: "Measuring and Extraction of Biological Information on New Handheld Biochip-Based Microsystem", IEEE TRANSACTIONS ON INSTRUMENTATION AND MEASUREMENT, IEEE SERVICE CENTER, PISCATAWAY, NJ, US, vol. 59, no. 1, 1 January 2010 (2010-01-01), pages 56-62, XP011280418, ISSN: 0018-9456, DOI: 10.1109/TIM.2008.2003321

## Description

### TECHNICAL FIELD

The present disclosure relates in general to biological analysis; and in particular to scalable, multi-channel high performance biosensing platforms, which are capable of high throughput biological analysis.

### BACKGROUND ART

Conventional biological analysis methods are being gradually replaced by sophisticated and compact biochip-based point-of-care devices. These biochip-based point-of-care devices perform common blood or glucose analysis at a fraction of the cost of conventional biological analysis methods. Also, biochip-based point-of-care devices typically require a much shorter analysis time compared to conventional biological analysis methods. Given the high potential of these biochip-based point-of-care technologies, research in biodetection methods and in biochips themselves, has significantly grown in the past decade.

From continued research in biodetection methods, new and more efficient biological analysis methods have become possible, providing a valuable tool for several applications, namely, but not limited to: clinical diagnostics, forensics, veterinarian, food industry, agriculture, and drug screening.

LOPES P ET AL: "Measuring and Extraction of Biological Information on New Handheld Biochip-Based Microsystem", IEEE TRANSACTIONS ON INSTRUMENTATION AND MEASUREMENT, IEEE SERVICE CENTER, PISCATAWAY, NJ, US, vol. 59, no. 1, 1 january 2010 (2010-01-01), pages 56-62,ISSN: 0018-945.6, DOI: 10:1109/TIM.2008.2003321 proposes techniques for the extraction of biological information in a recently developed handheld biochip-based microsystem. The microsystem is based on a magnetoresistive array biochip composed of a number of sensing sites. Experiments with the system in a setup to detect actual biological signals are presented.

### DISCLOSURE OF INVENTION

According to aspects of the present disclosure, a biological detection system is provided. The biological detection system is characterized by a biochip and a biosensing platform. The biochip has at least two blocks of sensors, each block of sensors having a signal acquisition channel that is independently accessible such that each block of sensors can be read in parallel. The biosensing platform has drive circuitry to drive each block of sensors of the biochip. In an exemplary implementation, the same or different driving signals can be applied to each block of sensors of the biochip. Also, the biosensing platform further has a set of acquisition interfaces arranged such that a select one acquisition interface is coupled to a corresponding one signal acquisition channel of the biochip. The biosensing platform also has a set of co-processors such that a select co-processor is uniquely associated with a corresponding one of the acquisition interfaces. Still further, the biosensing platform has a central controller that controls the drive circuitry. The central controller also interfaces with the set of acquisition interfaces. Still further, the central controller interfaces with the set of co-processors to read data from the biochip. Moreover, the biosensing platform may provide a direct user interface, a remote interface or combination thereof.

### BRIEF DESCRIPTION OF DRAWINGS

FIG. 1 is a schematic representation of a system for biological analysis that integrates a biochip and an embedded biosensing platform, according to aspects of the present disclosure;
FIG. 2A is an exploded view schematic representation of a biochip of the system for biological analysis illustrated in FIG. 1;
FIG. 2B is an exploded view schematic representation of a biosensing platform of the system for biological analysis illustrated in FIG. 1;
FIG. 2C is an exploded view schematic representation of an interface of the system for biological analysis illustrated in FIG. 1;
FIG. 3 is a block diagram of a biosensing platform, according to aspects of the present disclosure herein; and
FIG. 4 is a method of operating a biological detection system having a biochip and a biosensing platform.

### MODES FOR CARRYING OUT THE INVENTION

According to the present invention a system, is utilized to implement scalable, multi-channel high performance biosensing platforms, which are capable of high throughput biological analysis. For instance, aspects of the present disclosure find use in applications such as gene expression profiling, where thousands of biosensors analyze a given sample in parallel. In this regard, the biosensing platform described herein, integrates the complexity and computational needs of the electronic sensing system, into portable and standalone embedded systems with adequate computational capabilities for such high throughput analysis.

According to aspects of the present disclosure, systems are provided to deliver the computational power required by advanced biochip technology, and to allow simultaneous acquisition and processing at high sampling rates, through a high-performance and configurable acquisition and processing architecture. For instance, as will be described in greater detail herein, a biosensing platform is provided, which uses multiple dedicated co-processors performing Fast Fourier Transform (FFT)-based signal filtering, as well as other complex correction algorithms. An additional dedicated processor is used to synthesize the stimulus signals for the sensors, and a central processor is provided to control the whole system. With this approach, a fully autonomous embedded system is provided, which enables standalone operation of the platform, thus providing increased flexibility and usability.

### BIOLOGICAL DETECTION SYSTEM

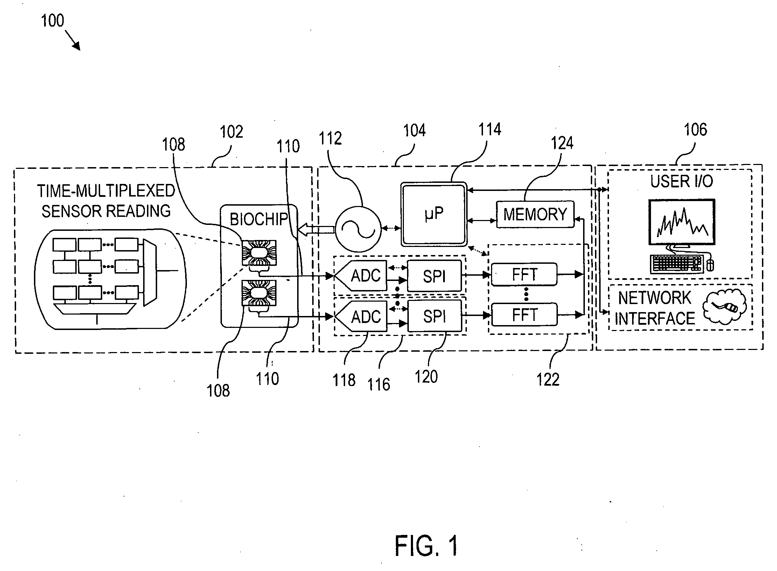

Referring now to the drawings, and in particular, to FIG. 1, a biological detection system 100 is illustrated in schematic form, which is capable of biological analysis according to aspects of the present disclosure. In general, the biological detection system 100 includes a biochip 102 and a biosensing platform 104.

The biological detection system 100 also integrates with an interface 106 to provide a complete lab-on-chip solution. The interface 106 may include for instance, local user interface peripherals (such as a keyboard, mouse, display, etc.), a remote interface, or a combination thereof, which can exchange data with electronics of the biosensing platform 104.

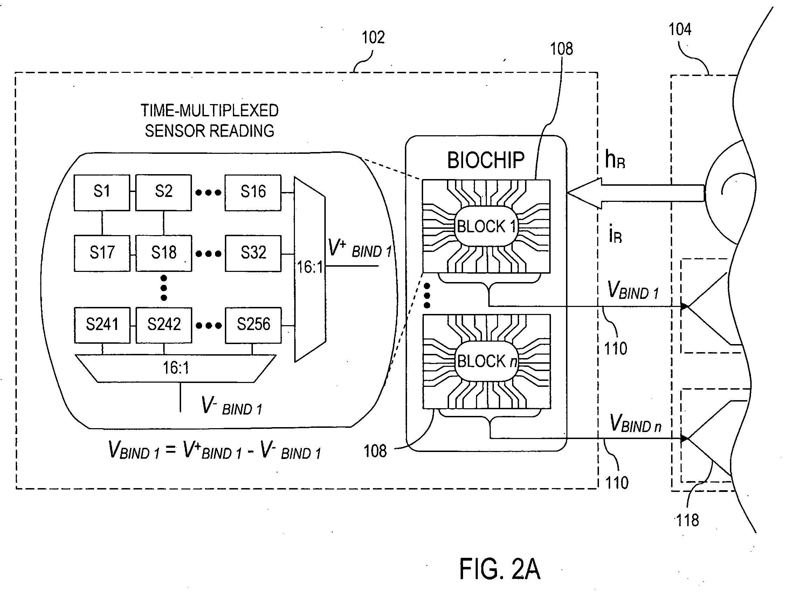

Referring generally to FIG. 1 and FIG. 2A, the biochip 102 (e.g., a magnetoresistive biochip) includes one or more "blocks" 108 of sensors. For instance, as illustrated, there are *n* blocks of sensors where *n* is any integer greater than zero. In this manner, particular blocks are referenced as 108-1, 108-2, ..., 108*-n,* etc. Each block 108 of sensors has a signal acquisition channel 110 that may be independently accessible such that each block 108 of sensors can be read in parallel, as will be described in greater detail herein. The biochip sensors (e.g., the blocks 108 of sensors) achieve a high sensitivity, high Signal-to-Noise Ratio (SNR) and high dynamic range when used in conjunction with the biosensing platform 104 described herein.

As best illustrated to the left of the biochip 102, an exploded view (of block 108-1 by way of example) illustrates that each block 108 includes a plurality of sensors. Each of the plurality of sensors can be of a various type (e.g. magnetic, temperature). By way of illustration, and not by way of limitation, there are 256 sensors in each block 108, arranged in an array of 16 rows and 16 columns. According to aspects of the present disclosure, more than 256 sensors are required for many important applications (e.g., gene expression profiling). To address this requirement, the illustrative biochip 102 combines CMOS with thin film fabrication technologies to provide the required number of sensors on a small biochip package. Thus, for instance, a biochip 102 with over 1,000 sensors is realized by implementing *n*=4 blocks 108, each with 256 sensors. However, in practice, each block 108 can have any number of sensors in its array. For instance, each block 108 can include 16 sensors or more in certain illustrative implementations.

In certain illustrative implementations, each block 108 of the biochip 102 may have a single acquisition channel. Each acquisition channel (or block of sensors themselves) can include integrated on-chip signal conditioning circuitry, and necessary readout circuitry to address and access the individual sensors of a corresponding block 108. For instance, by using a sensor reading technique, such as Time-Division Multiplexing (TDM), a control device is able to address each of the biosensors (256 biosensors in the above-example) through that single channel. For instance, each sensor can be read out individually using time-multiplexed reading techniques, such as by using a first multiplexer ("row multiplexer") to select a desired row, and a second multiplexer ("column multiplexer") to select a desired column. The sensor that is read out through the signal acquisition channel is thus the sensor at the intersection of the row selected by the row multiplexer and the column selected by the column multiplexer. In an illustrative implementation, the biochip 102 is characterized by each block of sensors having an array of at least 16 sensors and a corresponding time division multiplexer to read out individual sensors from a corresponding block. Moreover, in practical implementations, since there are multiple blocks 108 of sensors, the biochip 102 can provide a multichannel capability that includes up to, and/or in excess of 1000 sensors in certain exemplary implementations.

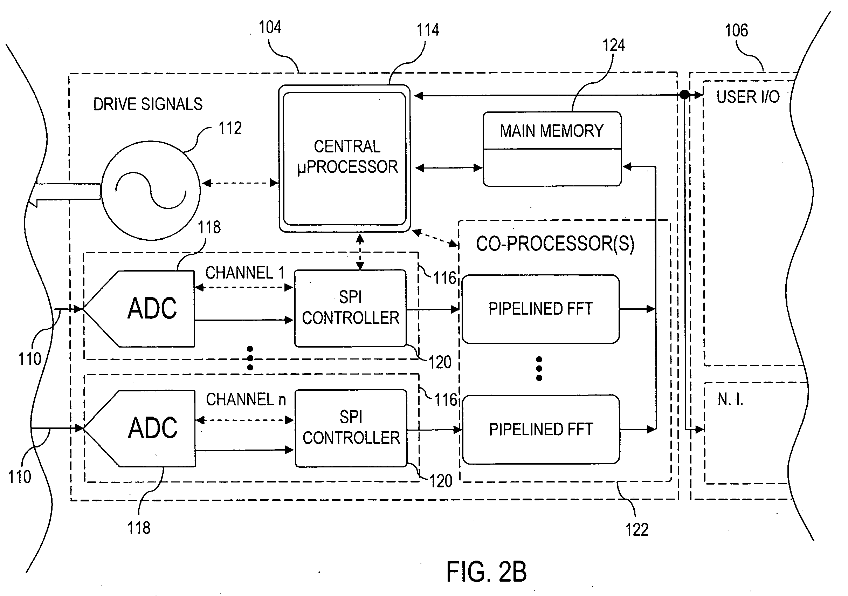

Referring now to FIG. 1 and FIG. 2B, the biosensing platform 104 includes in general, drive circuitry 112 that generates the necessary drive signal(s) for the corresponding biochip 102. More particularly, the biosensing platform 104 includes drive circuitry 112 to drive each block 108 of sensors of the biochip 102. In this regard, the manner in which the drive signals from the biosensing platform 104 couple to the biochip 102 will depend upon the particular implementation of the biochip 102. However, by way of example, the drive circuitry may include circuitry to generate bias currents and oscillating magnetic fields that are applied to the sensors of the biochip 102.

For instance, the drive circuitry 112 can include a single circuit with multiple outputs. Alternatively, the drive circuitry can include multiple independent circuits, e.g., an independent circuit for each block 108 of the corresponding biochip 102. Still further, the one or more circuits that make up the drive circuitry 112 can be software-based, Hardware based, or a combination of the above. In this regard, drive signals can be fully or partially programmable, controlled by set points, parameters or other program variables, made adjustable by control signals, e.g., programmed by control voltages or other control signals, etc.

As illustrated, the drive circuitry 112 is controlled by a central processor 114. Thus for example, in an illustrative implementation, the drive circuitry 112 is controlled by the central processor 114 to have an adjustable frequency. Moreover, the central processor 122 can control the drive circuitry to generate arbitrary signal shapes and amplitudes. For instance, the drive circuitry 112 can utilize Direct Digital Synthesizers (DDSs) that can be combined with arbitrary signal generators deployed in a reconfigurable fabric, which are controlled by the central processor 114, to provide the correct signals to the corresponding biochip 102.

The biosensing platform 104 also includes multiple (e.g., a set of) acquisition interfaces 116. As illustrated, the biosensing platform 104 includes *n* acquisition interfaces 116 where *n* is any integer greater than zero. In the illustrated example, a select one acquisition interface 116 is coupled to a corresponding one signal acquisition channel 110 of each block 108 of the biochip 102. Thus, where the biochip 102 has 4 blocks 108, each block having a single, signal acquisition channel 110, the corresponding biosensing platform 104 includes 4 acquisition interfaces 116, each acquisition interface 116 connected to a corresponding unique one of the biochip signal acquisition channels 110.

In the illustrative schematic representation, each acquisition interface 116 includes a converter 118, e.g., an analog to digital converter, which receives the signal from its corresponding signal acquisition channel 110. The analog to digital converter converts the sensor signal into a digital representation that can be further processed, as will be described in greater detail herein. As an illustrative example, an acquisition interface 116 is configured to measure sensor data from the corresponding signal acquisition channel 110 at a specific binding frequency or frequency band that is determined by the modulation induced by the magnetic field, the bias current, the sensor's response to these AC signals, and the system's noise and cross-talk. For instance, each analog to digital converter 118 may have a sampling frequency of at least 1 kHz and at least 12-bit resolution to accomplish the desired sampling.

The output of the analog to digital converter 118 couples to a controller 120, e.g., a serial peripheral interface controller. Each acquisition interface 116 may also include signal conditioning circuitry as necessary to interface with the analog signals from the biosensor 102. The controllers 120 are each controlled by the central processor 114.

Moreover, in an exemplary implementation, each controller 120 couples to a corresponding co-processor 122. In this manner, each controller 120 conveys information from its corresponding analog to digital converter 118 to its corresponding co-processor 122. Thus, the illustrated biosensing platform 104 includes a set of co-processors 122 (e.g.*, n* co-processors where *n* is any integer greater than zero) such that a select co-processor 122 is uniquely associated with a corresponding one of the acquisition interfaces 116. In alternative implementations, there need not be a one-to-one correspondence between acquisition interfaces 116 and co-processors 122. For instance, there may be a single co-processor 122 for all channels (acquisition interfaces 116). Also, a single co-processor 122 can be utilized to service a subset of acquisition interfaces 116.

Each dedicated co-processor 122 performs digital processing, e.g., Fast Fourier Transform (FFT)-based signal filtering on the data from the corresponding acquisition interface. Each co-processor 122 can also/alternatively perform other complex correction algorithms, e.g., a complex correction algorithm including a Kalman filter. Yet further, each co-processor 122 can be used to implement a temperature controller, a complex modulator and demodulator, etc. The co-processors 122 also communicate with the central processor 114, which controls the biosensing platform 104.

In this manner, to read data from the biochip, the central processor 114 of the biosensing platform 104 controls the drive circuitry 112. The central processor 114 also interfaces with the set of acquisition interfaces 116 (e.g., via the controllers 120). Yet further, the central processor 114 interfaces with the set of co-processors 122. Also, the central processor 114, a dedicated co-processor 122 or other processor (not shown) can be utilized to synthesize the stimulus signals for the data read from the sensors of the biochip 102.

The illustrated biosensing platform 104 further includes a main memory 124. The main memory can couple to the central processor 114 and/or to one or more of the co-processors 122. For instance, the central processor 114 and the co-processors 122 can share the main memory 124. In this regard, main memory 124 may be used to store programming, control information, calibration information, data, etc. As such, in certain applications, a computer processing device (e.g., personal computer or server computer) need not be connected to the biosensing platform 104 in order for the system to be operational. This allows, for instance, flexible standalone use. At a suitable time, the biosensing platform 104 can be coupled to the computer processing device, e.g., to download collected signal data, etc.

In this regard, the biosensing platform 104 includes electronic circuitry for addressing the biochip 102, for driving control signals and for conditioning and acquiring the biochip signals. The acquired signals are processed by a set of co-processors 122 and are transmitted to the central processor 114, e.g., which executes software that functions as a digital analyzer where, through the interface 106, e.g., a conventional graphical user-interface, the experiment is controlled by the operator or analyst. The biosensing platform 104 provides multiple signal acquisition channels 110 operating at a high sampling rate, thus producing a high volume of data. As such, the biosensing platform 104 also includes the necessary computation power that is required to handle the volume of data.

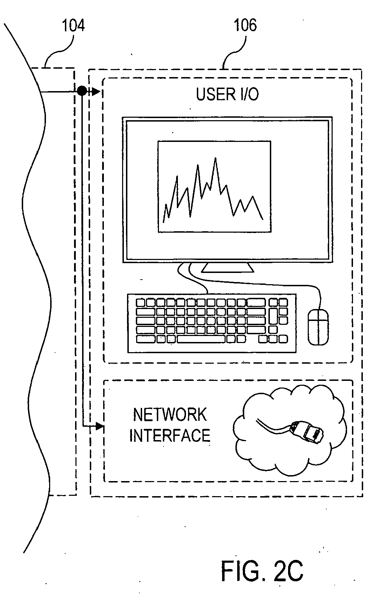

Referring to FIG. 1 and FIG. 2C, the interface 106 may comprise a monitor, keyboard, mouse or other computer peripherals, a graphical user interface, network interface, etc. In an illustrative implementation, the biological detection system includes a biosensing platform implemented in two components, including a programmable logic component and a processing system component, wherein the drive circuitry 112 (e.g., drive signal synthesizer), the co-processors 122 and the acquisition interfaces 116 are implemented in the programmable logic component, and the central processor 114 and the main memory 124 are implemented in the processing system.

The central processor 114 may further communicate over a network, which provides communications links between various processing devices, and may be supported by networking components that interconnect the processing devices, including for example, routers, hubs, firewalls, network interfaces, wired or wireless communications links and corresponding interconnections, cellular stations and corresponding cellular conversion technologies, e.g., to convert between cellular and tcp/ip, etc. Moreover, the network(s) may comprise connections using one or more intranets, extranets, local area networks (LAN), wide area networks (WAN), wireless networks (WIFI), the Internet, including the world wide web, cellular and/or other arrangements for enabling communication between the processing devices, in either real time or otherwise, e.g., via time shifting, batch processing, etc.

### Example Implementation

With reference to FIG. 1, and FIGS. 2A-2C generally, a laboratory assay begins by labeling biological targets under analysis with Magnetic Particles (MPs). Then, a bias current, *i_{B}*, and an oscillating magnetic field signal, *h_{B}*, are applied to the sensors of the biochip 102, e.g., via the drive circuitry 112 of the biosensing platform 104. Upon biomolecular recognition and washing, the sensors detect the fringe fields created by the MPs that have been captured by biological probes placed above the sensors. The biological information is then extracted by measuring the variations of the sensor voltage signal, *v_{bind}.*

To reduce the system cost and complexity, the sensors of each block 108 are measured using Time-Division Multiplexing (other modulation and multiplexing techniques can alternatively be applied). Since reaching a low noise level typically requires a high number of samples per sensor, biochip reading times can be slow, e.g., about 4 minutes. Such biomolecular recognition at a low speed, may be acceptable for certain biochips. However, such slow recognition does not scale when the number of sensors grows to 1000 or more. To circumvent issues of slow speeds, the biochip 102 is organized in groups of sensors, e.g., 256 sensor elements per block 108, and each sensor is time multiplexed within its corresponding block, as noted above. With this approach, the total reading time is constrained to the number of sensors within each group, as processing is performed in parallel. Thus biochip size can be scaled without increasing the overall reading time. Nevertheless, this also imposes significant hardware resources in the platform electronics. Each group 108 of the biochip 102 is serviced by a dedicated acquisition channel interface 116 of the biosensing platform 104, e.g., comprising signal conditioning circuits and an Analog-to-Digital Converter (ADC) 118. Furthermore, the data from each channel is processed in parallel, to allow the observation of the assay evolution in real-time.

Additional characteristics of the biochip must also be considered. According to an exemplary implementation, to avoid the effect of low frequency noise and maximize the attained SNR, the applied signals from the drive circuitry 112, e.g., applied signals is and *h_{B}* in the FIGURES, include an AC component. Consequently, *v_{bind}* is measured at a specific binding frequency, *f_{bind}*, which is determined by the modulation induced by the magnetic field *h_{B}* {*f₂*}*,* the bias *current i_{B}* {*f₁*}*,* the sensor's response to these AC signals, and the system's noise and cross-talk. At each sensor block multiple modulation frequencies can be applied in both magnetic field and bias current.

Due to the complexity of the biochip technology and of the circuitry that drives and measures the sensors, it may not be easy to determine a good *a-priori* estimate of the frequency at which the SNR is maximum. To optimize the biological sensitivity, *f_{bind}* should be adapted in real-time, by changing the frequencies of *i_{B}* and/or *h_{B}.* However, this introduces the added difficulty of determining the signal amplitude at an unknown frequency. According to illustrative aspects of the disclosure herein, a flexible and tractable method is to use an FFT of the signal acquired at each of the 1000+ sensors, to compute the signal amplitude *v_{bind}* at the frequency *f*_{*bind*.} Although it ensures a good adaptability, this approach may require a sampling frequency of at least 50 kHz and a frequency resolution as low as 1 Hz, for a 24-bit resolution in each sensor. That said, aspects of the present invention can function suitably, for instance, with a sampling frequency of at least 1 kHz and at least a 12-bit resolution. Regardless, this imposes large memory and hardware requirements, constraining the scalability of the system. Additionally, other complex correction algorithms can be implemented by each co-processor 122, namely but not limited to: drift correction, temperature controller, Kalman filters, complex modulator and/or demodulator (e.g. spread spectrum).

### ARCHITECTURE

The computational performance required by the biosensing platform 104 to ensure the resolution and reliability of bio-recognition assays can be achieved by optimizing the processors' architecture and by increasing the exploited parallelism with the usage of multicore structures. Nonetheless, it would be insufficient to exclusively address the performance limitations, since the bottleneck may also arise in the communication channels, such as the one connecting the acquisition circuits to the processing unit. Furthermore, due to the significant complexity of the underlying algorithms, the processing unit must be able to store and manipulate large datasets, making it clear that a high memory bandwidth and capacity are necessary to take full advantage of the enhanced processing capabilities.

As described more fully herein, the architecture to fulfill the requirements of the high throughput biosensing platform is depicted in FIG. 1. In general, when implementing the biosensing platform 104, a high-speed serial interface may be selected in order to provide a fast communication between the ADCs 118 and the set of dedicated co-processors 122 (e.g., cores) where the most computational intensive tasks are handled. All components, including the generator of the stimuli signals, are configured by a central processor 114, e.g., also referred to herein as a General Purpose Processor (GPP) which, aside from acting as the main system controller, can also perform auxiliary computations on the acquired data and user interface tasks as well as communication tasks. The central processor 114 shares its main memory 124 with the co-processors 122. A user interface, controlled through the central processor 114, is preferable performed through interface 106 which includes a set of I/O devices, such as the input peripherals used to control the experiment and a display on which the results are presented to the user. A network interface, controlled through the central processor 114, is also included, allowing the processed data to be transferred to an external database, easily available to other users.

### Central Processor and Main Memory

The central processor 114, e.g., the GPP, is a suitable device to configure the several system components, and thus the central processor 114 plays a key role on easing the system integration. In certain illustrative implementations, Operating System (OS) support is necessary, in order to benefit from the available software packages that provide complex system management functionalities and high-level interfaces to the standard I/O and memory peripherals. A processor-memory interconnection subsystem (subsystem that interconnects the central processor 114 to the main memory 124) can be equipped with DMA channels (or equivalent), allowing the central processor 114 to communicate with streaming peripherals within a shared memory space.

### High-Throughput Sensor Interface

Each acquisition channel is linked to one biochip sensor group 108 and directly feeds one specialized co-processor 122 with the data sampled by the ADC 118. In an exemplary implementation, since each ADC 118 can operate at a maximum rate of 96 Mbit/s, the absence of a high-speed data channel would seriously compromise the overall throughput. In the exemplary implementation, standard Low-Voltage Differential Signaling (LVDS) was chosen as a cost-effective and noise tolerant solution to achieve high transmission rates. On top of this physical layer, a Serial Peripheral Interface (SPI) 118 bus protocol is utilized, to provide configuration and addressing capabilities based on an SPI master/slave controller.

### Specialized Co-processors

The specialized co-processors 122 are responsible for measuring the amplitude of the sensors binding signal, *v_{bind},* from the biosensor 102, at a specific frequency. This signal is estimated using a zoom-in FFT approach together with other complex processing algorithms like Kalman filters that improve the overall data accuracy. In a first step, an FFT is performed with a low frequency resolution, and by using a subset of sensors in the same channel. This provides a coarse and preliminary identification of the best *f_{bind}* frequency to measure the *v_{bind,}* while keeping the hardware resources and power consumption at tolerable levels. After identifying the *fbind* range, band-pass filtering and signal demodulation are performed, to displace the frequency of interest down to base band. This allows the computation of multiple FFTs (one per channel) with a higher bit and frequency resolution, while still confining the required resources. Also, to increase the signal to noise ratio and achieve system scalability, the zoom-in procedure can be performed as many times as required. Complex demodulators (e.g. spread spectrum) can also be implemented in the co-processors to achieve a higher multiplexing capability.

Due to the involved computational complexity, a reconfigurable field programmable gate array (FPGA) device is regarded as a suitable technology to accommodate the multi-core infrastructure of specialized FFT co-processors, filters and demodulators 122. An FPGA combines the required high performance level, while still conferring the flexibility to change or update the adopted processing algorithms. In particular, multiple configurations can be stored in memory for each zoom-in level. In run-time, the user starts by observing the coarse signal spectrum of the initial subset of sensors. Then, the most favorable demodulation frequency and spectrum band is selected. This will trigger the FPGA reconfiguration and the deployment of the specialized co-processors in hardware, configured with the target demodulation frequency. At this point, the user can observe the binding signal for each sensor.

### I/O Peripherals and User Interface

A set of I/O peripherals 106 are utilized to make the biosensing platform 104 a standalone analysis and diagnosis system. These modules not only allow the user to take full control of the experiment, but also offer the visualization means for clinical interpretation purposes. The standard set of computer peripherals (i.e. USB keyboard / touch screen panel and HDMI monitor) is typically utilized as interfaces to the biosensing platform 104 to enhance the usability experience.

### Stimulus Signal Generator

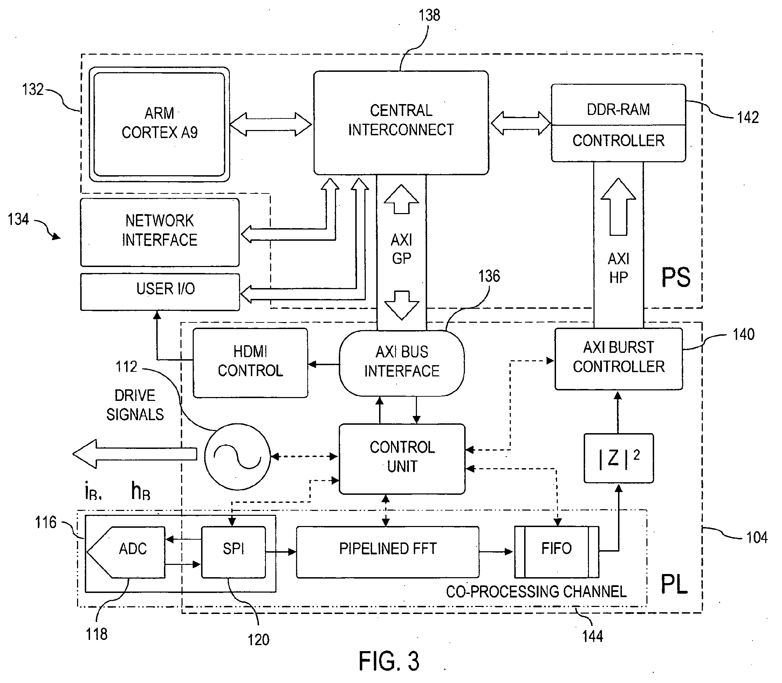

Referring now to FIG. 3, a portion of the biological detection system 100 of FIG. 1 is illustrated. In particular, FIG. 3 illustrates a partial view of the biosensing platform 104. For instance, only a single signal acquisition interface 116 is illustrated for convenience of illustration and discussion. Also, the biochip 102 and the interface devices 106 have been omitted for convenience of discussion.

Reference AC signals must be supplied by the drive generator 112 to the biosensors of the biochip 102, to produce a measurable response (e.g., *i_{B}* and *h_{B}* discussed above). The amplitude, shape and frequency ranges of these stimuli are partially constrained by the considered biochip technology. However, the noise level that the measurements are exposed to is inversely proportional to the frequency of these signals. Thus the accuracy of the results is strongly constrained by this parameter. As a consequence, a variable and configurable frequency generator component of the drive circuitry 112, which can be adjusted and parametrized by the central processor 114 (e.g., GPP), ensures the adaptability of the biosensing platform 104 to multiple generations of the biochip 102. The required signal generators can be implemented by using Direct Digital Synthesizers (DDSs) deployed in a reconfigurable fabric, which can be combined with additional hardware in the reconfigurable fabric to achieve complex modulation like spread spectrum, and controlled by the central processor 114.

### Exemplary Implementation

In a working exemplary embodiment, a Xilinx Zynq 7000 Extensible Processing Platform was utilized to build the biosensing platform 104 as an embedded system. This particular System on a Chip (SoC) includes a state of the art reconfigurable fabric and it offers most of the features required for the system described more fully with regard to FIGS. 1-3.

### Zynq SoC Architecture

The Zynq SoC integrates two independent modules in the same chip: a Processing System (PS), featuring a processor 132, e.g., a dual-core ARM Cortex-A9 processor, and Programmable Logic (PL), consisting of reconfigurable fabric compatible with Xilinx 7-Series FPGAs. In addition to the ARM processors and the associated DDR-memory controller, the PS also integrates a collection of industry-standard communication interfaces 134 (Gigabit Ethernet, USB, I2C and SPI). At boot time, the PS configures the PL by means of the Processor Configuration Access Port (PCAP) and initializes the system clocks. Through the PCAP, the ARM processor is able to exploit the software-driven dynamic partial reconfiguration capabilities of the reconfigurable fabric, thus promoting an adapted and optimized usage of the reconfigurable part of this SoC.

The PS-PL interconnection can be achieved by using an interface, e.g., the Advanced extensible Interface (AXI) bus protocol, through any of the provided connectivity ports, namely, General-Purpose (AXI-GP), High-Performance (AXI-HP), and Accelerator Coherency Port (AXI-ACP). For instance, as illustrated, two master and two slave AXI-GP interfaces 136 connect the PL to a PS central interconnect 138, allowing for low throughput data and control transfers. When high throughput is required, a separate interface 140, e.g., an AXI-HP interface, connects directly to the main memory 142. In this interface 140, read and write operations are mediated by an asynchronous 1 kB FIFO with a configurable data width of 32 or 64 bits. The AXI-ACP interface connects the PL directly to the Snoop Control Unit (SCU), to ensure data coherency at the L2 shared cache of the ARM processors. All these ports can be individually enabled and configured. On the PL side, these interfaces are implemented using the existing reconfigurable logic, thus having an associated cost in terms of hardware resources.

### Implementation

While the ARM processor on the PS is used to control the platform, the reconfigurable fabric on the PL is used to implement the dedicated co-processors and to support the interface with the biochip. The base software layer running in the PS is implemented in an exemplary implementation, using the Linaro Ubuntu GNU/Linux OS, which provides optimized tools and software packages for ARM SoCs. By adopting this OS, the PS provides a rich interface to the user by using the existing set of computer peripherals.

FIG. 3 depicts the block diagram of the implemented architecture, considering a single-channel 116. It corresponds to the initial zoom-in phase, where an FFT is computed over a small subset of sensors from a single acquisition channel to determine the binding frequency, *f_{bind}* that maximizes the signal to noise ratio. In the multi-channel implementation, where parallel channels read biological information of 1000+ sensors, the Co-processor channel 1X in Fig. 3 is replicated as necessary, according to the blocks 108/acquisition channels 116 of the biochip 102.

Several AXI interfaces connect the reconfigurable fabric to the ARM processor and to the associated main memory, either for control or for high throughput data transfers. By following the design strategy commonly adopted in DMA-based peripherals, each devised co-processor provides a slave interface for configuration purposes, along with a master controller that writes in the shared memory bus. Data consistency between the dedicated co-processors and the GPP is achieved by using a contiguous region in the main memory, marked as non-cacheable and excluded from the system's memory management. The OS is able to read this memory region by remapping the region into the kernel virtual address space, thus allowing it to be easily accessed by a custom device driver.

### FFT Co-processor

In an illustrative implementation, the FFT co-processors (e.g., co-processor 122) in the PL are based on the Xilinx FFT IP core v7.1, and adopt a pipeline operation to produce an output per clock cycle in a stream-based processing scheme. The cores are configured to receive 24-bit fixed-point real inputs, although a higher precision is used inside the cores to decrease numerical error propagation between intermediate stages. Furthermore, embedded DSP blocks are used in the butterfly computations to allow for a better balance in the DSP/BRAM usage within the available reconfigurable fabric. A significant amount of BRAMs is needed for data and phase factor storage when the FFTs are computed with a high amount of points. However, the adoption of the zoom-in approach allows the total memory requirements per channel, to be kept at reasonable levels. In all zoom-in levels, the synchronous reset and start bit signals are obtained from a control register, allowing for an operation fully controlled by the PS. Also, since the considered signal processing algorithm requires the squaring of the FFT complex output, an additional squaring block is introduced at FIFO output.

### PL-PS Data Streaming and Interface

In an illustrative implementation, the communication between the several logic blocks on the reconfigurable fabric and the ARM GPP is ensured by specifically defined control and data channels. By following a memory-mapped I/O approach, a set of control registers are made accessible to the programs executing on the ARM, providing convenient software reset and start flags triggering functionalities. These registers are placed on an AXI bus, mastered by the CPU through an AXI-GP interface port. An AXI-HP port is used to transfer the FFT output frames directly to the main memory, where they can be subsequently accessed by the CPU. Since this shared memory region is contiguous and is not directly managed by the OS kernel (as explained above), an AXI Burst Master Controller is used to provide these co-processing channels 144 with a direct memory access. This solution provides a data-transfer performance equivalent to a conventional DMA engine, but reduces the hardware requirements by approximately 7 times.

When multichannel processing is considered, the architecture in FIG. 3 is expanded to incorporate as many co-processing blocks as the number of channels, e.g., blocks 108/acquisition channels 116 of the biochip 102 (dashed box labeled Co-processing channel 144 in the lower part of Fig. 3). The co-processing blocks are then connected to the AXI-HP port, which provides a communication channel between the co-processors and the main memory capable of supporting data transfers of up to 1.2 GByte/s (estimate). This is well above the 72 KBit/s bit-rate of the ADC in certain biosensor applications. Even considering that higher bit-rates are required for the disclosed biosensing platform, this bandwidth is enough to support multiple channels (the biochips 102 comprise 4 channels), and still provides enough resources for future expansions. However, it should be noticed that a continuous data transmission must be assured to achieve a throughput of 1.2 GByte/s. To guarantee this requisite, a dual clock domain FIFO memory is used to implement a bridge between the FFT processing channels and the AXI-HP bus connected to the main memory.

### System I/O and User Interface

In an illustrative implementation, two different interfaces are supported by the biosensing platform: i) an SPI-based interconnection with each channel ADC and with stimuli generator, ii) the interface with the human operator. The connections to the biosensing ADC and with the stimuli generator can be accomplished by an open-source core implementation of the SPI protocol, available at OpenCores.org. The selection of this particular core takes into account its particular characteristics in terms of versatility and resource efficiency.

Referring generally to FIG. 1, FIG. 2C, and FIG. 3, in an illustrative implementation, the interface 106, e.g., graphical user interface, is accomplished by using an HDMI graphics port and an USB keyboard, USB touch-screen panel or other suitable interface. Although an HDMI interface already exists in the Zynq platform, the controller has to be implemented on the reconfigurable fabric. The controller module was obtained from a reference design targeting an HDMI output interface, along with the physical and data streaming blocks required to handle DMA video streams. These DMA engines are connected to two separate AXI-HP ports, enabling them to directly gather video data from the main memory. The HDMI control logic (also on the PL side), is connected to an AXI-GP interface and is mapped into the memory-space. of the OS, allowing the usage of existing software device drivers.

### Method Calibrating a Biological Detection System

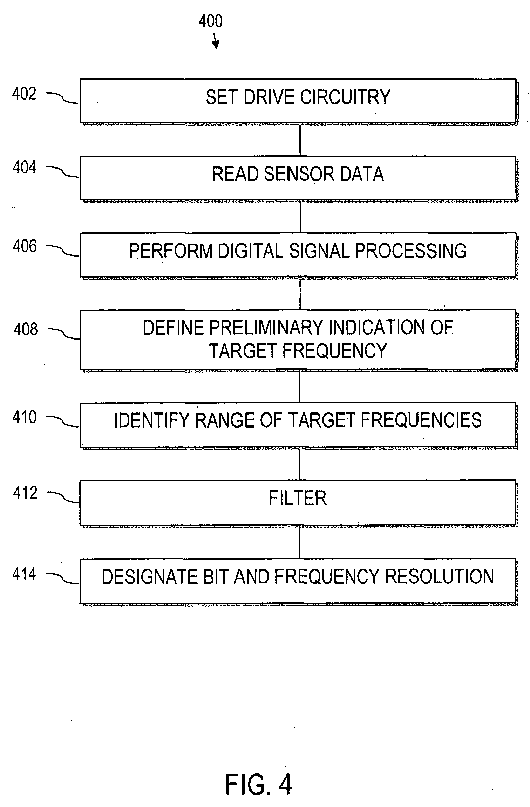

Referring to FIG. 4, a method 400 of operating a biological detection system having a biochip may be used, for instance, to calibrate drive circuitry signals for the best signal to noise ratio, by setting, drive circuitry to generate a set of drive signals and reading, sensor data from a biochip. The method may perform digital processing, with a low frequency resolution on sensor data extracted from the biochip, using a subset of sensors that access a common signal acquisition channel of the biochip. The method may use the result of the digital processing with a low frequency resolution to define at, a preliminary indication of a target frequency to measure a sensor data output from the signal acquisition channel, and by identifying, a range or band of target frequencies. Yet further, the method may perform, filtering and signal demodulation to displace a frequency (band) of interest from the identified range of target frequencies down to a base band and may designate, a bit and frequency resolution from the base band that maximizes the signal to noise ratio of the data read from the signal acquisition channel.

The method may perform demodulation where sensor data extracted from the biochip results from using modulation techniques. Performing digital processing may be achieved by performing a fast Fourier transform with a low frequency resolution on sensor data extracted from the biochip, using a subset of sensors that access a common signal acquisition channel of the biochip. Complex correction algorithms including at least one of a: Kalman filter, drift compensation, and a temperature controller, on the result from the Fourier transform, may be used.

### Experimental Results

To properly evaluate the scalable multichannel architecture, experimental results were obtained by using a ZedBoard featuring a Zynq XC7Z020, one of the smallest and less power demanding Zynq SoCs, and was synthesized and mapped using the Xilinx 14.3 development tools. The presented analysis starts by considering a single channel architecture implementation with the largest allowed FFT core, which is used to determine, with maximum accuracy, the biosensors binding frequency region. Experimental results are presented in Table 1 and show that the FFT-size (64k samples) is limited by the amount of available block RAMs in the PL. Furthermore, the results show that the resources required by the remaining structure corresponds to only 33.5% of the available LUTs, only 8 BRAMs and 19 DSPs. From this, it can be concluded that the majority of the PL hardware resources are made available to be used by the co-processing channel(s).

**Table 1. System resources for a single channel 64K FFT.**

| Component | LUT (53200) | | BRAM (140) | | DSP48 (220) | |
|---|---|---|---|---|---|---|
| 64k FFT core | 7157 | (13.5%) | 106 | (75.7%) | 103 | (46.8%) |
| Squaring module | 149 | (0.3%) | 0 | - | 16 | (7.2%) |
| Drive signals (DDS) | 174 | (0.3%) | 2 | (1.4%) | 3 | (1.4%) |
| AXI wrapper | 572 | (1.1%) | 0 | - | 0 | - |
| SPI Master | 131 | (0.2%) | 0 | - | 0 | - |
| Core system | 13857 | (26.0%) | 6 | (4.3%) | 0 | - |
| Full system | 25097 | (47.2%) | 114 | (82.1%) | 122 | (55.4%) |

To evaluate the scalability and adaptability of the architecture to the case when zoom-in is performed and multiple channels are required, bit-streams with smaller FFT cores were generated and the number of channels was maximized by balancing the usage of DSPs, BRAMs and LUTs. Table 2 presents the resources used by a single co-processing channel 144, considering FFTs with increasing number of points, and the maximum number of co-processing channels 144 supported on the PL. It should be noticed that for the 1k-, 2k- and 4k-FFT the maximum number of channels is constrained by the number of available DSP48 elements; for larger FFTs the limitation lies in the BRAMs. Also, in the 32k-FFT, to support 2 channels, some block RAMs were implemented using LUTs.

**Table 2. Co-processing channel hardware requirements.**

| FFT size (#samples) | Hardware resources (per channel) | | | | | | Maximum #channels |
|---|---|---|---|---|---|---|---|
| | LUT (53200) | | BRAM (140) | | DSP48 (220) | | |
| 1024 | 3686 | (7%) | 8 | (6%) | 24 | (11%) | 8 |
| 2048 | 4375 | (8%) | 11 | (8%) | 31 | (14%) | 6 |
| 4096 | 4765 | (9%) | 14 | (10%) | 31 | (14%) | 6 |
| 16384 | 7201 | (14%) | 28 | (20%) | 40 | (18%) | 4 |
| 32768 | 7916 | (15%) | 55 | (39%) | 49 | (22%) | 2 |
| 65536 | 7254 | (14%) | 106 | (76%) | 103 | (47%) | 1 |

The presented results suggest that it is possible to achieve a multichannel processing system composed of 8 FFT channels of 1024 points. Hence, with up to 256 biosensors per channel, a total of 2,048 biosensors can be processed and the sweep time is reduced by a factor of 8. Additional multiplexing can be achieved by using other complex modulation techniques like multi carriers or spread spectrum. This demonstrates the adaptability of the proposed system to multichannel processing, while keeping the required amount of hardware resources compatible with this entry level Zynq SoC. The FFT cores are capable of operating above 180 MHz, producing 1 output sample per clock cycle. Considering that the system must support an ADC sample rate of up to 4 Msps, which corresponds to the state of the art in high resolution converters, this suggests that the FFT cores do not impose any limitation to the signal analysis performance.

On the other hand, the AXI-HP allows an estimated maximum transfer rate of 1.2 GByte/s, while, for a data width of 32 bits, the FFT core outputs a bit-rate of at most 128 Mbit/s. Hence, the AXI-HP bandwidth does not limit the system operation, supporting multiple acquisition channels. Considering that the developed biochip features 4 sensor blocks (1024 sensors), the bit rate could still be increased if faster ADCs become available.

From these results, it can be concluded that the architecture herein, mapped on a Zynq SoC, provides the computational resources required to implement a fully embedded and high performance biosensing platform. Furthermore, the implemented prototype also demonstrated that a scalable and adaptable platform can be obtained with the existing reconfiguration capabilities, capable of adapting to biochips with higher sensor count and different computational needs.

### Miscellaneous

According to aspects of the present disclosure, systems are provided to deliver the computational power required by advanced biochip technology, and to allow simultaneous acquisition and processing at high sampling rates, through a high-performance and configurable acquisition and processing architecture. For instance, as described in greater detail herein, a biosensing platform is provided, which uses multiple dedicated co-processors performing Fast Fourier Transform (FFT)-based signal filtering, as well as other complex correction and modulation/multiplexing algorithms. An additional dedicated processor is used to synthesize the stimulus signals for the sensors, and a central processor is provided to control the whole system. With this approach, a fully autonomous embedded system is provided, which enables standalone operation of the platform, thus providing increased flexibility and usability.

In this manner, new high density biochips are regarded as highly promising solutions for numerous applications, where high throughput analysis is required. However, existing electronic platforms are not able to acquire and process the higher amount of produced data. The scalable multi-channel and high-throughput architecture herein meets the needs to acquire and process this higher amount of data, e.g., even when implemented on a single Zynq SoC. For instance, even when using one of the smallest Zynq's, experimental results demonstrate that the system supports 8 times more sensors and a 1000+ times higher sampling frequency than the previous generation of the biosensing platform. By taking advantage of the reconfigurable characteristics of this SoC, results also suggest that the chosen platform allows to implement a scalable system supporting co-processors with configurable FFT sizes, enabling optimized and adjustable signal measurement for each biochip, without sacrificing efficiency in hardware resource usage, allowing the developed system to be expanded in order to deal with the requirements of future biochips.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

## Claims

1. A biological detection system (100), comprising:
a biochip (102) having at least two blocks (108) of sensors, each block of sensors having a signal acquisition channel (110) that is independently accessible such that each block of sensors can be read in parallel; and
a biosensing platform (104) having:
drive circuitry (112) to drive each block (108) of sensors of the biochip;
a set of acquisition interfaces (116) arranged such that a select one acquisition interface (116) is coupled to a corresponding one signal acquisition channel (110);
charaterised by a set of co-processors (122) such that a select co-processor is uniquely associated with a corresponding one of the acquisition interfaces (116); and
a central processor (114) that controls the drive circuitry (112), interfaces with the set of acquisition interfaces (116) and interfaces with the set of cho-processors (122), to read data from the biochip.

2. The biological detection system of claim 1, wherein the biochip is further **characterized by**:
each block of sensors having an array of at least 16 sensors and a corresponding time division multiplexer to read out individual sensors from a corresponding block.

3. The biological detection system according to any of the preceding claims, wherein the set of acquisition interfaces is further **characterized by**:
an analog to digital converter (118) and a controller (120), where the controller conveys information from the analog to digital converter to its corresponding co-processor.

4. The biological detection system of claim 3, wherein the analog to digital converter has a sampling frequency of at least 1 kHz and at least 12-bit resolution.

5. The biological detection system according to any preceding claim, wherein each co-processor is **characterized by**:
a configuration that performs at least one of:
Fast Fourier Transform (FFT)-based signal filtering on the data from its corresponding acquisition interface;
a complex correction algorithm including a Kalman filter,
a temperature controller; and
a complex modulator and demodulator.

6. The biological detection system according to any preceding claim, further **characterized by**:
a main memory that is shared by the central processor and each of the co-processors.

7. The biological detection system according to any preceding claim, wherein the drive circuitry is further **characterized by**:
circuitry to generate bias currents and oscillating magnetic fields that are applied to the sensors of the biochip.

8. The biological detection system according to any preceding claim, **characterized by**:
the drive circuitry is coupled to the central processor such that a signal generator of the drive circuitry is controlled by the central processor to have an adjustable frequency and can also generate arbitrary signal shapes and amplitudes.

9. The biological detection system according to any preceding claim, wherein the drive circuitry is **characterized by**:
Direct Digital Synthesizers (DDSs) that can be combined with arbitrary signal generators deployed in a reconfigurable fabric, which are controlled by the central processor.

10. The biological detection system according to any of the preceding claims, wherein an acquisition interface is configured to measure sensor data from the corresponding signal acquisition channel at a specific binding frequency or frequency band that is determined by the modulation induced by the magnetic field, the bias current, the sensor's response to these AC signals, and the system's noise and cross-talk.

11. The biological detection system according to any of the preceding claims, **characterized by**:
the biosensing platform implemented in two components, including a programmable logic component and a processing system component, wherein the drive circutiry, the co-processors and the acquisition interfaces are implemented in the programmable logic component, and the central processor and the main memory are implemented in the processing system.

## Patentansprüche

1. Biologisches Detektionssystem (100), umfassend:
einen Biochip (102) mit mindestens zwei Blöcken (108) von Sensoren, wobei jeder Block von Sensoren einen Signalerfassungskanal (110) aufweist, der unabhängig zugreifbar ist, so dass jeder Sensorblock parallel ausgelesen werden kann; und
eine Biosensing-Plattform (104), die aufweist:
eine Treiberschaltung (112), um jeden Block (108) von Sensoren des Biochips zu betreiben;
einen Satz von Erfassungsschnittstellen (116), der so angeordnet ist, dass eine ausgewählte Erfassungsschnittstelle (116) mit einem korrespondierenden Signalerfassungskanal (110) verbunden ist;
**gekennzeichnet durch** einen Satz von Koprozessoren (122) solcherart, dass ein ausgewählter Koprozessor eindeutig einer korrespondierenden der Erfassungsschnittstellen (116) zugeordnet ist; und
einen Zentralrechner (114), der die Treiberschaltung (112) steuert, mit dem Satz von Erfassungsschnittstellen (116) über Schnittstellen verbunden ist und mit dem Satz von Koprozessoren (122) über Schnittstellen verbunden ist, um die Daten von dem Biochip auszulesen.

2. Biologisches Detektionssystem nach Anspruch 1, wobei der Biochip außerdem **dadurch gekennzeichnet ist, dass**:
jeder Sensorblock eine Anordnung von mindestens 16 Sensoren und einen zugehörigen Zeitmultiplexer aufweist, um einzelne Sensoren aus einem entsprechenden Block auszulesen.

3. Biologisches Detektionssystem gemäß einem der vorangehenden Ansprüche, worin der Satz von Erfassungsschnittstellen außerdem **gekennzeichnet ist durch**:
einen Analog-Digital-Wandler (118) und eine Steuereinheit (120), wobei die Steuereinheit Informationen von dem Analog-Digital-Wandler zu seinem zugehörigen Koprozessor übermittelt.

4. Biologisches Detektionssystem nach Anspruch 3, worin der Analog-Digital- Wandler eine Abtastfrequenz von mindestens 1 kHz und eine Auflösung von mindestens 12 Bit aufweist.

5. Biologisches Detektionssystem gemäß einem der vorhergehenden Ansprüche, worin jeder Koprozessor **gekennzeichnet ist durch**:
eine Konfiguration, die mindestens eines davon ausführt:
Schnelle Fourier-Transformations (FFT)-basierte Signalfilterung der Daten der zugehörigen Erfassungsschnittstelle;
einen komplexen Korrekturalgorithmus, umfassend einen Kalman-Filter,
eine Temperatursteuereinheit; und
einen komplexen Modulator und Demodulator.

6. Biologisches Detektionssystem gemäß einem der vorangehenden Ansprüche, weiter **gekennzeichnet durch**:
einen Hauptspeicher, der von dem Zentralrechner und jedem der Koprozessoren gemeinsam genutzt wird.

7. Biologisches Detektionssystem gemäß einem der vorangehenden Ansprüche, worin die Treiberschaltung weiterhin **gekennzeichnet ist durch**:
eine Schaltung, um Vorströme und oszillierende Magnetfelder zu erzeugen, welche an die Sensoren des Biochips angelegt werden.

8. Biologisches Detektionssystem gemäß einem der vorangehenden Ansprüche, **dadurch gekennzeichnet, dass**:
die Treiberschaltung so mit dem Zentralrechner verbunden ist, dass ein Signalgenerator der Treiberschaltung durch den Zentralrechner gesteuert wird, um eine regelbare Frequenz zu ermöglichen, und außerdem beliebige Signalformen und Amplituden erzeugen kann.

9. Biologisches Detektionssystem gemäß einem der vorangehenden Ansprüche, worin die Treiberschaltung **gekennzeichnet ist durch**:
Direkte Digitale Synthetisierer (DDSs), die mit beliebigen Signalgeneratoren, formiert in einem rekonfigurierbaren Gefüge, kombiniert werden können, welche **durch** den Zentralrechner gesteuert werden.

10. Biologisches Detektionssystem gemäß einem der vorangehenden Ansprüche, worin eine Erfassungsschnittstelle zum Messen von Sensordaten aus dem zugehörigen Signalerfassungskanal bei einer/m spezifischen Bindungsfrequenz oder Frequenzband konfiguriert ist, welche/welches durch die Modulation bestimmt wird, die durch das Magnetfeld, den Vorstrom, das Ansprechen des Sensors auf diese AC-Signale und den Rausch- und Übersprecheffekt des Systems induziert wird.

11. Biologisches Detektionssystem gemäß einem der vorangehenden Ansprüche, **gekennzeichnet durch**:
die Biosensing-Plattform, die in zwei Komponenten realisiert ist, umfassend eine programmierbare logische Komponente und eine Verarbeitungssystem-Komponente, wobei die Treiberschaltung, die Koprozessoren und die Erfassungsschnittstellen in der programmierbaren logischen Komponente implementiert sind und der Zentralrechner und der Hauptspeicher in dem Verarbeitungssystem implementiert sind.

## Revendications

1. Système de détection biologique (100), comprenant :
une biopuce (102) ayant au moins deux blocs (108) de capteurs, chaque bloc de capteurs ayant un canal d'acquisition de signaux (110) qui est indépendamment accessible de telle sorte que chaque bloc de capteurs peut être lu en parallèle ; et
une plateforme de biodétection (104) ayant :
une circuiterie de pilotage (112) pour piloter chaque bloc (108) de capteurs de la biopuce ;
un ensemble d'interfaces d'acquisition (116) agencées de telle sorte qu'une interface d'acquisition sélectionnée (116) est couplée à un canal d'acquisition de signaux correspondant (110) ;
**caractérisée par** un ensemble de coprocesseurs (122) de telle sorte qu'un coprocesseur sélectionné est associé de manière unique à l'une correspondante des interfaces d'acquisition (116) ; et
un processeur central (114) qui commande la circuiterie de pilotage (112), s'interface avec l'ensemble d'interfaces d'acquisition (116) et s'interface avec l'ensemble de coprocesseurs (112), pour lire des données en provenance de la biopuce.

2. Système de détection biologique selon la revendication 1, dans lequel la biopuce est en outre **caractérisée par** :
chaque bloc de capteurs ayant un réseau d'au moins 16 capteurs et un multiplexeur à répartition dans le temps correspondant pour lire des capteurs individuels provenant d'un bloc correspondant.

3. Système de détection biologique selon l'une quelconque des revendications précédentes, dans lequel l'ensemble d'interfaces d'acquisition est en outre **caractérisé par** :
un convertisseur analogique-numérique (118) et un contrôleur (120), le contrôleur transportant des informations du convertisseur analogique-numérique à son coprocesseur correspondant.

4. Système de détection biologique selon la revendication 3, dans lequel le convertisseur analogique-numérique possède une fréquence d'échantillonnage d'au moins 1 kHz et une résolution d'au moins 12 bits.

5. Système de détection biologique selon une quelconque revendication précédente, dans lequel chaque coprocesseur est **caractérisé par** :
une configuration qui réalise au moins l'un parmi :
un filtrage de signaux basé sur une Transformée de Fourier Rapide (FFT) sur les données en provenance de son interface d'acquisition correspondante ;
un algorithme de correction complexe comprenant un filtre de Kalman,
un contrôleur de température ; et
un modulateur et un démodulateur complexes.

6. Système de détection biologique selon une quelconque revendication précédente, **caractérisé en outre par** :
une mémoire principale qui est partagée par le processeur central et chacun des coprocesseurs.

7. Système de détection biologique selon une quelconque revendication précédente, dans lequel la circuiterie de pilotage est en outre **caractérisée par** :
une circuiterie pour générer des courants de polarisation et des champs magnétiques oscillants qui sont appliqués aux capteurs de la biopuce.

8. Système de détection biologique selon une quelconque revendication précédente, **caractérisé par** :
la circuiterie de pilotage est couplée au processeur central de telle sorte qu'un générateur de signaux de la circuiterie de pilotage est commandé par le processeur central pour avoir une fréquence ajustable et peut également générer des formes et des amplitudes de signaux arbitraires.

9. Système de détection biologique selon une quelconque revendication précédente, dans lequel la circuiterie de pilotage est **caractérisée par** :
des Synthétiseurs Numériques Directs (DDS) qui peuvent être combinés à des générateurs de signaux arbitraires déployés dans une matrice reconfigurable, qui sont commandés par le processeur central.

10. Système de détection biologique selon l'une quelconque des revendications précédentes, dans lequel une interface d'acquisition est configurée pour mesurer des données de capteur en provenance du canal d'acquisition de signaux correspondant à une fréquence de liaison spécifique ou une bande de fréquences qui est déterminée par la modulation induite par le champ magnétique, le courant de polarisation, la réponse du capteur à ces signaux à courant alternatif, et le bruit et la diaphonie du système.

11. Système de détection biologique selon l'une quelconque des revendications précédentes, **caractérisé par** :
la plateforme de biodétection implémentée dans deux composants, comprenant un composant de logique programmable et un composant de système de traitement, dans laquelle la circuiterie de pilotage, les coprocesseurs et les interfaces d'acquisition sont implémentés dans le composant de logique programmable, et le processeur central et la mémoire principale sont implémentés dans le système de traitement.
